# EUROPÄISCHE PATENTANMELDUNG

(11) **EP 2 774 563 A1**
(43) Veröffentlichungstag der Anmeldung: **10.09.2014**
(21) Anmeldenummer: 13158368.4
(22) Anmeldetag: 08.03.2013
(51) Int. Cl.: A61B 17/70

(54) **Wirbelsäulenimplantat zur Stabilisierung und Versteifung von Wirbelkörpern einer Wirbelsäule**

(71) Anmelder: Spinelab AG, 8406 Winterthur (CH)
(72) Erfinder: Clark, Jonathan, 8302 Kloten (CH); Zehnder, Thomas, 8806 Bäch (CH)
(74) Vertreter: BOVARD AG

(57) **Zusammenfassung**

Ein Wirbelsäulenimplantat (1) zur Stabilisierung und Versteifung von Wirbelkörpern einer Wirbelsäule umfasst erste Knochenschrauben (7), zweite Knochenschrauben (2), erste starre Verbindungselemente (10), die in die ersten Knochenschrauben (7) einsetzbar und darin befestigbar sind, zweite Verbindungselemente (5), die jeweils aus einem elastischen Stab (6) bestehen, der in die zweiten Knochenschrauben einsetzbar und darin befestigbar sind. Über Kupplungsmittel (11) können jeweils ein erstes Verbindungselement (10) und ein zweites Verbindungselement (5) miteinander verbunden werden. Diese Kupplungsmittel (11) umfassen ein Tragteil (26), der mit einem zapfenförmigen Vorsprung (28) versehen ist, dessen Aussenform mindestens teilweise der Aussenform des elastischen Stabes (6) entspricht. Der zapfenförmige Vorsprung (28) ist in den einen Teil der U-förmigen Aufnahme (12) einer zweiten Knochenschraube (2) einsetzbar und fixierbar, in den anderen Teil der U-förmigen Aufnahme (12) ist der Endbereich eines elastischen Stabes (6) einsetzbar und fixierbar.

## Beschreibung

Die vorliegende Erfindung bezieht sich auf ein Wirbelsäulenimplantat zur Stabilisierung und Versteifung von Wirbelkörpern einer Wirbelsäule, umfassend erste Knochenschrauben mit ersten Aufnahmemitteln, zweite Knochenschrauben mit zweiten Aufnahmemitteln, erste Verbindungselemente, die starr sind und in die ersten Aufnahmemittel der ersten Knochenschrauben einsetzbar und darin befestigbar sind, zweite Verbindungselemente, die jeweils aus einem elastischen Stab bestehen und die in eine U-förmige Aufnahme der zweiten Aufnahmemittel der zweiten Knochenschrauben einsetzbar und darin mittels Spannmitteln befestigbar sind, welche U-förmige Aufnahme mit ersten Strukturen versehen sind, und Kupplungsmitteln, mit welchen jeweils ein erstes Verbindungselement und ein zweites Verbindungselement miteinander verbindbar sind.

Derartige Wirbelsäulenimplantate sind in vielfältiger Weise bekannt. Mit diesen Wirbelsäulenimplantaten wird erreicht, dass bei der Wirbelsäule bereichsweise eine starre Stabilisierung der Wirbelkörper ermöglicht wird, während in anderen Bereichen die Wirbelkörper durch die elastische Ausgestaltung des Systems gestützt und stabilisiert werden, ohne dass eine Versteifung erfolgt. Im Bereich der starren Stabilisierung der Wirbelkörper ist erwünscht, dass ein knöchernes Verwachsen der betroffenen und stabilisierten Wirbelkörper erreicht wird, bei der elastischen Stabilisierung soll keine Versteifung der Wirbelkörper auftreten.

Ein derartiges Wirbelsäulenimplantat zur Stabilisierung und Versteifung von Wirbelkörpern einer Wirbelsäule ist beispielsweise aus der EP A 1 961 392 bekannt.

Wenn nach einer gewissen Zeit das Zusammenwachsen und die Verknöcherung der Wirbelkörper miteinander, die durch das Wirbelsäulenimplantat versteift wurden, soweit erfolgt ist, dass die Eigenstabilität gegeben ist, werden die versteifenden Bereiche des Wirbelsäulenimplantats nicht mehr benötigt, sie sind eher überflüssig und können stören. Es ist deshalb sinnvoll, wenn diese versteifenden Bereiche des Wirbelsäulenimplantats nach dem entsprechenden Verwachsungsvorgang vom elastisch stabilisierenden Bereich vollständig entkoppelt oder gegebenenfalls sogar entfernt werden können, während die Bereiche des Wirbelsäulenimplantats, die der Stabilisierung der Wirbelkörper dienen, beibehalten werden sollen, um diese unterstützende und stabilisierende Wirkung weiter ausüben zu können. Hierzu können der versteifende Bereich und der stabilisierende und unterstützende Bereich des Wirbelsäulenimplantats voneinander entkoppelt werden. Eine derartige Einrichtung ist beispielsweise aus der EP A 2 436 325 bekannt.

Die Aufgabe der vorliegenden Erfindung besteht nunmehr darin, ein Wirbelsäulenimplantat zur Stabilisierung und Versteifung von Wirbelkörpern einer Wirbelsäule derart zu verbessern, dass der Aufbau eines derartigen Wirbelsäulenimplantats vereinfacht wird und dadurch eine vereinfachte Handhabung beim Einsetzen dieses Wirbelsäulenimplantats in die Wirbelsäule eines Körpers erreichbar ist und eine spätere Entkoppelung des elastischen Bereichs und des starren Bereichs ebenfalls in einfachster Weise möglich wird.

Erfindungsgemäss erfolgt die Lösung dieser Aufgabe dadurch, dass die Kupplungsmittel ein Tragteil umfassen, welches mit einem ersten Bereich, der mit einem zapfenförmigen Vorsprung versehen ist, dessen Aussenform mindestens teilweise der Aussenform des elastischen Stabes entspricht und der mit zweiten Strukturen versehen ist, die komplementär zu den ersten Strukturen in der U-förmigen Aufnahme ausgebildet sind, welcher zapfenförmige Vorsprung in den einen Teil der U-förmigen Aufnahme einer zweiten Knochenschraube einsetzbar und über die Spannmittel fixierbar ist, und in den anderen Teil der U-förmigen Aufnahme ein Endbereich eines elastischen Stabes einsetzbar und über die Spannmittel fixierbar ist, und mit einem dem ersten Bereich gegenüberliegenden zweiten Bereich versehen ist, der mit weiteren Spannmitteln ausgestattet ist, in welchen ein Endbereich eines ersten Verbindungselementes fixierbar ist.

Mit dieser Ausgestaltung kann der Tragteil der Kupplungsmittel in gleicher Weise in die U-förmige Aufnahme der zweiten Knochenschraube eingesetzt werden, wie der entsprechende Stab, Tragteil und elastischer Stab können in einfachster Weise über die Spannmittel in der zweiten Knochenschraube fixiert werden, was eine einfache Handhabung ergibt. Zum Entkoppeln des elastischen Stabes und der ersten Verbindungselemente können die Spannmittel gelöst werden, der Tragteil kann aus der U-förmigen Aufnahme der zweiten Knochenschraube herausgehoben werden, ohne dass der in dieser Knochenschraube gehaltene elastische Stab manipuliert werden muss, die gleichen Spannmittel können danach wieder auf die Knochenschraube aufgesetzt werden.

In vorteilhafter Weise ist die U-förmige Aufnahme der zweiten Aufnahmemittel aus zwei einander gegenüberliegenden Schenkel gebildet, welche innenseitig mit einem Gewinde ausgestattet sind, wodurch ein sehr einfacher Aufbau dieser Aufnahmemittel erreicht wird.

In vorteilhafter Weise sind die Spannmittel aus einer Spannschraube gebildet, welche in das Gewinde zwischen die beiden Schenkel einschraubbar ist, was eine sehr einfache Handhabung für einen Chirurgen zur Folge hat.

In vorteilhafter Weise ist zwischen der Spannschraube und dem Stab beziehungsweise den zapfenförmigen Vorsprung, welche in die U-förmige Aufnahme eingesetzt sind, ein Presselement angeordnet, was eine Schonung des Stabes und des zapfenförmigen Vorsprungs beim Spannen mit der Spannschraube ermöglicht.

Die gegen den Stab beziehungsweise den zapfenförmigen Vorsprung gerichtete Oberfläche des Presselements ist ebenfalls mit zweiten Strukturen ausgestattet, die zu einer besseren formschlüssigen Verbindung der entsprechenden Elemente beiträgt.

Eine weitere vorteilhafte ausgestaltung der Erfindung besteht darin, dass die Oberflkäche der zweiten Verbindungselemente mindestens teilweise mit zweiten Strukturen versehen sind. Diese zweiten Strukturen sind komplementär zu den ersten Strukturen ausgebildet, wodurch sich eine gute formschlüssige Verbindung ergibt.

In vorteilhafter Weise sind die ersten Strukturen und die zweiten Strukturen als Rippen und Rillen ausgebildet, die quer zu Längsachse des in die U-förmige Aufnahme eingesetzten Stabes verlaufen, was einen optimalen Formschluss ergibt und eine einfache Herstellung dieser Strukturen ermöglicht.

Eine weitere vorteilhafte Ausgestaltung der Erfindung besteht darin, dass die weiteren Spannmittel aus einer im zweiten Bereich des Tragteils angeordneten Ausnehmung bestehen, in welche der Endbereich eines ersten Verbindungselementes einsetzbar und über eine weitere Spannschraube fixierbar ist, was ebenfalls zu einem einfachen Aufbau der Einrichtung beiträgt.

Eine weitere vorteilhafte Ausgestaltung der Erfindung besteht darin, dass der Endbereich des ersten Verbindungselementes, der in die Ausnehmung des Tragteils einsetzbar ist, kugelförmig ausgebildet ist, und dass der in der Ausnehmung angeordnete Abstützbereich für den Endbereich des ersten Verbindungselementes kugelkalottenförmig ausgebildet ist. Dadurch wird erreicht, dass das erste Verbindungselement, das starr ist, bezüglich dem Tragteil und somit bezüglich der zweiten Knochenschraube, in welcher dieser Tragteil fixiert ist, allseitig in einem gewissen Winkelbereich ausgerichtet werden kann und in optimaler Weise an die in die Wirbelkörper eingesetzten Knochenschrauben angepasst werden kann, wodurch erreicht wird, dass ein nicht exaktes Setzen der Knochenschrauben in die Wirbelkörper statt durch aufwändiges Verbiegen des starren Verbindungselementes während des Eingriffs durch die Möglichkeit einer polyaxialen Ausrichtung des ersten Verbindungselementes ausgeglichen werden kann.

In vorteilhafter Weise sind das erste Verbindungsmittel, die ersten und zweiten Knochenschrauben und die Kupplungsmittel aus metallischen Legierungen, insbesondere Titanlegierungen, gebildet, wodurch die erforderliche Stabilität erreicht wird.

In vorteilhafter Weise sind die zweiten Verbindungselemente aus einem biokompatiblen Kunststoff gebildet, wodurch die gewünschte Elastizität erhalten werden kann.

Eine Ausgestaltung der Erfindung wird nachfolgend anhand der beiliegenden Zeichnungen beispielhaft näher erläutert.

Es zeigt:
Figur 1 in räumlicher Darstellung ein Wirbelsäulenimplantat, das ein erstes starres Verbindungselement und ein zweites elastisches Verbindungselement aufweist, welche über in einer zweiten Knochenschraube gehaltene Kupplungsmittel verbunden sind;
Figur 2 eine Längsschnittdarstellung des Wirbelsäulenimplantats gemäss Figur 1; und
Figur 3 eine Explosionsdarstellung des Wirbelsäulenimplantats gemäss Figur 1 und 2.

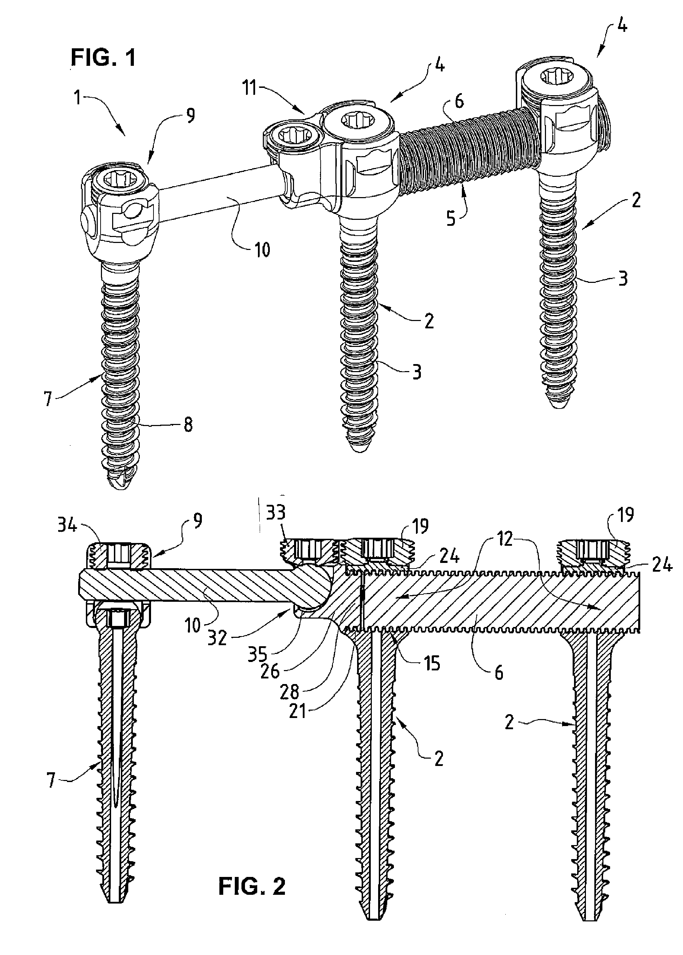

Wie aus Fig. 1 ersichtlich ist, setzt sich ein erfindungsgemässes Wirbelsäulenimplantat 1 zur Stabilisierung und Versteifung von Wirbelkörpern einer Wirbelsäule aus zweiten Knochenschrauben 2 zusammen, die jeweils aus einem mit einem Gewinde versehenen Einschraubteil 3 und aus zweiten Aufnahmemitteln 4 bestehen. In bekannter Weise ist eine derartige zweite Knochenschraube 2 mit einem Einschraubteil 3 in einen Wirbelkörper einer Wirbelsäule einschraubbar. In die zweiten Aufnahmemittel 4 ist ein zweites Verbindungselement 5 eingesetzt und festgehalten. Dieses zweite Verbindungselement 5 ist als elastischer Stab 6 ausgebildet, welcher aus einem elastischen Material, beispielsweise aus einem biokompatiblen Kunststoff auf Polyurethan-Basis besteht.

Das erfindungsgemässe Wirbelsäulenimplantat 1 umfasst ferner erste Knochenschrauben 7, welche ebenfalls in bekannter Weise jeweils einen Einschraubteil 8 aufweisen, der dem Einschraubteil 3 der zweiten Knochenschraube 2 entspricht und entsprechend in den vorgesehenen Wirbelkörper der Wirbelsäule einschraubbar ist. Diese ersten Knochenschrauben 7 sind mit ersten Aufnahmemitteln 9 ausgestattet, in welche ein erstes Verbindungselement 10, das stangenförmig ausgebildet ist, eingesetzt und befestigt werden kann. Dieses erste Verbindungselement 10 ist starr und besteht aus einem metallischen Werkstoff, beispielsweise einer Titanlegierung.

Das jeweilige erste Verbindungselement 10 ist jeweils mit einem daran angrenzenden zweiten Verbindungselement 5 über Kupplungsmittel 11 verbindbar. Diese Kupplungsmittel 11 sind lösbar in einer zweiten Knochenschraube 2 gehalten, in welcher ein Endbereich des elastischen Stabes 6 ebenfalls gehalten ist, wie später im Detail noch beschrieben wird. In die Kupplungsmittel 11 kann der Endbereich eines ersten Verbindungsmittels 10 eingesetzt und fixiert werden, wie ebenfalls später noch im Detail beschrieben wird.

Ein derartiges Wirbelsäulenimplantat 1 ist in praktisch beliebiger Weise an die zu stabilisierende Wirbelsäule anpassbar. Dieses Wirbelsäulenimplantat kann aus starren ersten Verbindungselementen 10 und elastischen zweiten Verbindungselementen 5 zusammengesetzt werden, die mit den jeweiligen ersten bzw. zweiten Knochenschrauben 7 bzw. 2 zusammenwirken. Die jeweiligen Verbindungselemente 5 und 10 können auf das gewünschte Mass abgelängt werden und durch die entsprechenden Kombinationen kann die Wirbelsäule in den gewünschten Bereichen elastisch stabilisiert und in anderen gewünschten Bereichen versteift werden, wobei in die vorgesehenen Wirbelkörper jeweils die entsprechende Knochenschraube eingeschraubt wird.

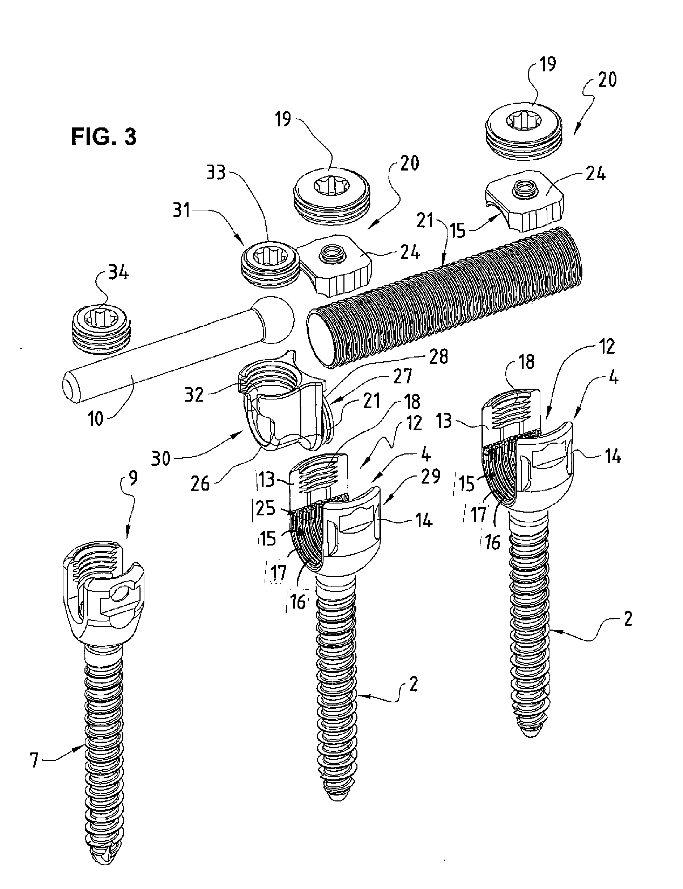

Wie aus Fig. 3 ersichtlich ist, umfassen die zweiten Knochenschrauben 2 zweite Aufnahmemittel 4. Diese weisen eine U-förmige Aufnahme 12 auf, die durch zwei einander gegenüberliegende Schenkel 13 und 14 gebildet wird. Die U-förmige Aufnahme ist mit ersten Strukturen 15 versehen, die im vorliegenden Ausführungsbeispiel als Rippen 16 und Rillen 17 ausgebildet sind. Innenseitig sind die beiden Schenkel 13 und 14 mit einem Gewinde 18 ausgestattet, in welche die als Spannschraube 19 ausgebildeten Spannmittel 20 eingesetzt werden können.

In die U-förmige Aufnahme 12 der jeweiligen zweiten Knochenschrauben 2 kann ein elastischer Stab 6 eingesetzt werden, der das zweite Verbindungselement 5 bildet. Dieser elastische Stab 6 ist in diesem Ausführungsbeispiel an der Oberfläche mit zweiten Strukturen 21 ausgestattet, die im hier dargestellten Ausführungsbeispiel ebenfalls als Rippen 22 und Rillen 23 ausgebildet sind. Beim Einsetzen in die U-förmige Aufnahme 12 gelangen die Rippen und Rillen 22 und 23 des elastischen Stabes 6 in die Rillen 17 und Rippen 16 der U-förmigen Aufnahme 12, man erhält dadurch eine formschlüssige Verbindung, die eine achsiale Verschiebung des elastischen Stabes 6 in der U-förmigen Aufnahme 12 ausschliessen. Selbstverständlich könnten die ersten Strukturen 15 und die zweiten Strukturen 21 auch in geeigneter Form anders ausgebildet sein, so dass ebenfalls eine formschlüssige Verbindung entsteht.

Selbstverständlich können auch elastische Stäbe 6 eingesetzt werden, die beispielsweise eine glatte Oberfläche aufweisen. Durch die Elastizität dringen beim Spannen des jeweiligen elastischen Stabes 6 in die U-förmige Aufnahme 12 die ersten Strukturen 15 in die Oberfläche des elastischen Stabes 6 ein, wodurch auch hier eine eigentliche formschlüssige Verbindung entsteht.

Wenn der elastische Stab 6 in die U-förmige Aufnahme 12 der jeweiligen zweiten Knochenschraube 2 eingesetzt ist, können die Spannmittel 20 angebracht werden. Diese Spannmittel 20 umfassen zusätzlich ein Presselement 24, das zwischen die Spannschraube 19 und den elastischen Stab 6 eingesetzt werden kann. Auf der dem elastischen Stab 6 zugewandten Seite kann dieses Presselement 24 ebenfalls mit ersten Strukturen 15 ausgestattet werden, wodurch eine verbesserte Verbindung entsteht.

Bei der in Fig. 3 in der Mitte dargestellten zweiten Knochenschraube 2 ist in den einen Teil 25 der U-förmigen Aufnahme 12 ein Tragteil 26 eingesetzt. Dieser Tragteil 26 weist an einem ersten Bereich 27 ein zapfenförmiger Vorsprung 28 auf, dessen Aussenform im wesentlichen der Aussenform des elastischen Stabes 6 entspricht. Dieser zapfenförmige Vorsprung 28 ist ebenfalls mit zweiten Strukturen 21 ausgestattet. Somit kann dieser zapfenförmige Vorsprung 28 in den einen Teil 25 der U-förmigen Aufnahme 12 eingesetzt werden und über die Spannmittel 20 fixiert werden, wodurch dieser zapfenförmige Vorsprung 28 formschlüssig in der U-förmigen Aufnahme 12 der zweiten Knochenschraube 2 gehalten wird. In den anderen Teil 29 der U-förmigen Aufnahme 12 der zweiten Knochenschraube 2 kann ein Endbereich des elastischen Stabes 6 eingesetzt und ebenfalls über die Spannmittel 20, wie vorgängig beschrieben worden ist, fixiert werden. Somit lässt sich der Tragteil 26 und der Endbereich des elastischen Stabes 6 über das eine Presselement 24 und die eine Spannschraube 19 in einfacher Weise in der zweiten Knochenschraube 2 formschlüssig halten.

Der zweite Bereich 30 des Tragteils 26, der dem ersten Bereich 27 gegenüberliegend ist, ist mit weiteren Spannmitteln 31 ausgestattet, in welchen ein Endbereich eines ersten Verbindungselementes 10 fixierbar ist.

Im zweiten Bereich 30 des Tragteils 26 ist eine Ausnehmung 32 angeordnet, in welche der Endbereich des ersten Verbindungselementes 10 einsetzbar ist. Diese Ausnehmung 32 ist mit einem Gewinde 33 ausgestattet, in welches Gewinde eine weitere Spannschraube 33 eingeschraubt werden kann. Mit dieser weiteren Spannschraube 33 lässt sich der Endbereich des zweiten Verbindungselementes 10 im Tragteil 26 fixieren.

Das erste Verbindungselement 10 wird in bekannter Weise in die ersten Aufnahmemittel 9 der ersten Knochenschraube 7 eingesetzt, und kann über eine Spannschraube 34 in der ersten Knochenschraube 7 fixiert werden.

Fig. 2 zeigt eine Schnittdarstellung des zusammengesetzten Wirbelsäulenimplantats 1. In Fig. 2 linksseitig angeordnet ist eine erste Knochenschraube 7, in deren ersten Aufnahmemittel 9 über die Spannschraube 34 ein erstes starres Verbindungselement 10 gehalten ist. Der der ersten Knochenschraube 7 abgewandte Endbereich des ersten starren Verbindungselementes 10 ist in die Ausnehmung 32 des Tragteils 26 eingesetzt. Dieser Endbereich des ersten Verbindungselementes 10 ist, wie auch aus Fig. 3 ersichtlich ist, kugelförmig ausgebildet. Der entsprechende in der Ausnehmung 32 angeordnete Abstützbereich 35, gegen welchen dieser kugelförmige Endbereich durch die weitere Spannschraube 33 gespannt wird, ist entsprechend kugelkalottenförmig ausgebildet. Durch diese Ausgestaltung erhält man eine optimale Fixierung des Endbereichs des ersten Verbindungselementes 10 im Tragteil 26, zudem kann das erste Verbindungselement 10 bezüglich des Tragteils 26 winklig ausgerichtet werden, wodurch in bekannter Weise eine einfachere Handhabung erreicht wird.

Der zapfenförmige Vorsprung 28 mit den zweiten Strukturen 21 ist in die U-förmige Aufnahme 12 der zweiten Knochenschraube 2 eingesetzt. Durch die in dieser U-förmigen Aufnahme 12 angeordneten ersten Strukturen 15 wird dieser zapfenförmige Vorsprung 28 formschlüssig darin gehalten. Ebenfalls in die U-förmige Aufnahme 12 eingesetzt ist der eine Endbereich des elastischen Stabes 6, der in diesem Ausführungsbeispiel ebenfalls mit zweiten Strukturen 21 versehen ist, und welche ebenfalls in die ersten Strukturen 15 in der U-förmigen Aufnahme 12 formschlüssig gehalten sind. Der zapfenförmige Vorsprung 28 und der Endbereich dieses elastischen Stabes 6 werden durch die Spannschraube 19 und das dazwischen angeordnete Presselement 24 in die U-förmige Aufnahme 12 gepresst.

Der elastische Stab 6 ist in eine weitere zweite Knochenschraube 2 eingesetzt und wird darin über die Spannschraube 19 und das eingesetzte Presselement 24 formschlüssig gehalten.

Wenn nun beispielsweise nach der Versteifung der Wirbelkörper, die durch den starren Bereich des Wirbelsäulenimplantats fixiert worden waren, dieser starre Teil des Wirbelsäulenimplantats entfernt werden soll, lässt sich dies in optimaler Weise erreichen. Das erste Verbindungselement 10 wird durch Lösen der Spannschrauben 33 und 34 herausgenommen, die Spannschraube 19 der zweiten Knochenschraube 2 wird gelöst und mit dem Presselement 24 aus der zweiten Knochenschraube herausgenommen, der Tragteil 26 kann aus der zweiten Knochenschraube 2 herausgehoben werden, ohne dass die formschlüssige Verbindung des elastischen Stabes 6 in der zweiten Knochenschraube 2 beeinträchtigt wird. Nach dem Wegnehmen des Tragteils 26 kann der Pressteil 24 und die Spannschraube 19 wieder auf die zweite Knochenschraube aufgesetzt und gespannt werden, die Verbindung zwischen elastischem Stab und zweiter Knochenschraube ist wieder in optimaler Weise hergestellt.

In einer zweiten Knochenschraube 2 können auch jeweils ein Endbereich von zwei elastischen Stäben 6 eingesetzt und fixiert werden. Dadurch könnte man zwei elastische Stäbe 6 in einer zweiten Knochenschraube 2 in optimaler Weise verbinden. Dies hätte den Vorteil, dass kürzere elastische Stäbe 6 eingesetzt werden könnten, was die Herstellung dieser elastischen Stäbe vereinfachen würde.

Dieses erfindungsgemässe Wirbelsäulenimplantat kann in vielfältiger Weise in Wirbelsäulen von Körpern eingesetzt werden, einerseits ist die Handhabung sehr einfach, andererseits ist insbesondere die Möglichkeit der Entkopplung gegeben, die ebenfalls in sehr einfacher Weise vorgenommen werden kann.

## Patentansprüche

1. Wirbelsäulenimplantat zur Stabilisierung und Versteifung von Wirbelkörpern einer Wirbelsäule, umfassend erste Knochenschrauben (7) mit ersten Aufnahmemitteln (9), zweite Knochenschrauben (2) mit zweiten Aufnahmemitteln (4), erste Verbindungselemente (10), die starr sind und in die ersten Aufnahmemittel (9) der ersten Knochenschrauben (7) einsetzbar und darin befestigbar sind, zweite Verbindungselemente (5), die jeweils aus einem elastischen Stab (6) bestehen und die in eine U-förmige Aufnahme (12) der zweiten Aufnahmemittel (4) der zweiten Knochenschrauben (2) einsetzbar und darin mittels Spannmitteln (20) befestigbar sind, welche U-förmige Aufnahme (12) mit ersten Strukturen (15) versehen sind, und Kupplungsmitteln (11), mit welchen jeweils ein erstes Verbindungselement (10) und ein zweites Verbindungselement (5) miteinander verbindbar sind, **dadurch gekennzeichnet, dass** die Kupplungsmittel (11) ein Tragteil (26) umfassen, welches mit einem ersten Bereich (27), der mit einem zapfenförmigen Vorsprung (28) versehen ist, dessen Aussenform mindestens teilweise der Aussenform des elastischen Stabes (6) entspricht und der mit zweiten Strukturen (21) versehen ist, die komplementär zu den ersten Strukturen (15) in der U-förmigen Aufnahme (12) ausgebildet sind, welcher zapfenförmige Vorsprung (28) in den einen Teil (25) der U-förmigen Aufnahme (12) einer zweiten Knochenschraube (2) einsetzbar und über die Spannmittel (20) fixierbar ist, und in den anderen Teil (29) der U-förmigen Aufnahme (12) ein Endbereich eines elastischen Stabes (6) einsetzbar und über die Spannmittel (20) fixierbar ist, und mit einem dem ersten Bereich (27) gegenüberliegenden zweiten Bereich (30) versehen ist, der mit weiteren Spannmitteln (31) ausgestattet ist, in welchen ein Endbereich eines ersten Verbindungselementes (10) fixierbar ist.

2. Wirbelsäulenimplantat zur Stabilisierung und Versteifung von Wirbelkörpern einer Wirbelsäule nach Anspruch 1, **dadurch gekennzeichnet, dass** die U-förmige Aufnahme (12) der zweiten Aufnahmemittel (4) aus zwei einander gegenüberliegenden Schenkeln (13, 14) gebildet ist, welche innenseitig mit einem Gewinde (18) ausgestattet sind.

3. Wirbelsäulenimplantat zur Stabilisierung und Versteifung von Wirbelkörpern einer Wirbelsäule nach Anspruch 2, **dadurch gekennzeichnet, dass** die Spannmittel (20) aus einer Spannschraube (19) gebildet sind, welche in das Gewinde (18) zwischen die beiden Schenkel (13, 14) einschraubbar ist.

4. Wirbelsäulenimplantat zur Stabilisierung und Versteifung von Wirbelkörpern einer Wirbelsäule nach Anspruch 3, **dadurch gekennzeichnet, dass** zwischen der Spannschraube (19) und dem Stab (6) bzw. dem zapfenförmigen Vorsprung (28), welche in die U-förmige Aufnahme (12) eingesetzt sind, ein Presselement (24) angeordnet ist.

5. Wirbelsäulenimplantat zur Stabilisierung und Versteifung von Wirbelkörpern einer Wirbelsäule nach Anspruch 4, **dadurch gekennzeichnet, dass** die gegen den Stab (6) bzw. den zapfenförmigen Vorsprung (28) gerichtete Oberfläche des Presselements (24) ebenfalls mit ersten Strukturen (15) ausgestattet sind.

6. Wirbelsäulenimplantat zur Stabilisierung und Versteifung von Wirbelkörpern einer Wirbelsäule nach einem der Ansprüche 1 bis 5, **dadurch gekennzeichnet, dass** die ersten Strukturen (21) und die zweiten Strukturen (15) als Rippen (16; 22) und Rillen (17; 23) ausgebildet sind, die quer zur Längsachse des in die U-förmige Aufnahme (12) eingesetzten Stabes (6) verlaufen.

7. Wirbelsäulenimplantat zur Stabilisierung und Versteifung von Wirbelkörpern einer Wirbelsäule nach einem der Ansprüche 1 bis 6, **dadurch gekennzeichnet, dass** die Oberflkäche der zweiten Verbindungselemente (5) mindestens teilweise mit zweiten Strukturen (21) versehen sind.

8. Wirbelsäulenimplantat zur Stabilisierung und Versteifung von Wirbelkörpern einer Wirbelsäule nach einem der Ansprüche 1 bis 7, **dadurch gekennzeichnet, dass** die weiteren Spannmittel (31) aus einer im zweiten Bereich (30) des Tragteils (26) angeordneten Ausnehmung (32) bestehen, in welche der Endbereich eines ersten Verbindungselements (10) einsetzbar und über eine weitere Spannschraube (33) fixierbar ist.

9. Wirbelsäulenimplantat zur Stabilisierung und Versteifung von Wirbelkörpern einer Wirbelsäule nach Anspruch 8, **dadurch gekennzeichnet, dass** der Endbereich des ersten Verbindungselementes (10), der in die Ausnehmung (32) des Tragteils (26) einsetzbar ist, kugelförmig ausgebildet ist, und dass der in der Ausnehmung (32) angeordnete Abstützbereich (35) für den Endbereich des ersten Verbindungselementes (10) kugelkalottenförmig ausgebildet ist.

10. Wirbelsäulenimplantat zur Stabilisierung und Versteifung von Wirbelkörpern einer Wirbelsäule nach einem der Ansprüche 1 bis 9, **dadurch gekennzeichnet, dass** die ersten Verbindungselemente (10), die ersten und zweiten Knochenschrauben (7, 2) und die Kupplungsmittel (11) aus metallischen Legierungen, insbesondere Titanlegierungen gebildet sind.

11. Wirbelsäulenimplantat zur Stabilisierung und Versteifung von Wirbelkörpern einer Wirbelsäule nach einem der Ansprüche 1 bis 10, **dadurch gekennzeichnet, dass** die zweiten Verbindungselemente (5) aus einem biokompatiblen Kunststoff gebildet sind.
